(19) Europäisches Patentamt
European Patent Office
Office européen des brevets

(11) **EP 1 748 081 A1**

(12) **EUROPEAN PATENT APPLICATION**

(43) Date of publication:
**31.01.2007 Bulletin 2007/05**

(51) Int Cl.:
*C12Q 1/68* (2006.01)  *H05B 6/80* (2006.01)
*H05B 6/02* (2006.01)  *H05B 6/10* (2006.01)
*H05B 3/78* (2006.01)

(21) Application number: **05106980.5**

(22) Date of filing: **28.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicants:
• **Roche Diagnostics GmbH**
  **68305 Mannheim (DE)**
  Designated Contracting States:
  **DE**
• **F. HOFFMANN-LA ROCHE AG**
  **4070 Basel (CH)**
  Designated Contracting States:
  **AT BE BG CH CY CZ DK EE ES FI FR GB GR HU IE IS IT LI LT LU LV MC NL PL PT RO SE SI SK TR**

(72) Inventors:
• **Sandoz, Roger**
  **6330 Cham (CH)**
• **Calasso, Irio Giuseppe, Dr.**
  **6415 Arth (CH)**
• **Kopp, Martin, Dr.**
  **6332 Hagendorn (CH)**

(74) Representative: **Rauscher, Andreas**
  **Roche Diagnostics GmbH**
  **Patentabteilung**
  **Sandhofer Strasse 116**
  **D-68305 Mannheim (DE)**

(54) **Analytical method and device**

(57) A method is provided that conveniently allows rapid amplification of nucleic acids. Two different heat sources are used to heat the reaction mixture.

FIG 4

P [W] for 95°C bead-temperature and 5mg of beads in water of constant 50°C

EP 1 748 081 A1

**Description**

Field of the invention

Subject of the present invention is a method for amplifying nucleic acids, a method for treating a liquid, an apparatus for heating a liquid in a vessel, and a system for heating a liquid in a vessel.

Background of the invention

The invention is particularly useful in the field of health care, where reliable analysis of samples for components contained therein is needed. Chemical reactions needing heating are well known, for example from Molecular Diagnostics, where nucleic acids are known to denature, i.e. to become single stranded from a hybrid of two strands, by applying heat above the melting temperature of the hybrid.

A method that uses reactions cycles including such denaturation step is the polymerase chain reaction (PCR). This technology has revolutionized the field of nucleic acid treatment, particularly the analysis of nucleic acids, by providing a tool to increase the amount of nucleic acids of a particular sequence from negligible to detectable amounts. PCR is described in EP 0 201 184 and EP 0 200 362.

Methods for heating a composition of matter are also known. For example in US 2002/0061588 there is described a method for heating a nucleic acid by attaching it to a nanoparticle and applying energy to this nanoparticle. By the heat, nucleic acid hybrids on the surface of the modulator are denatured and one of the strands can dissociate into the surrounding liquid. However, this method is quite inefficient regarding heating and amplification.

In US 2004/0129555 there is described a method for heating a mixture containing a dye using a pulsed LASER. In US 6633785 there is described a method for heating a micro-tube using either resistance heating or inductive heating.

The prior art methods did not provide temperature conditions to efficiently amplify nucleic acids.

Brief description of the drawings

Figure 1 shows three possible ways of immobilizing primer on the solid particles (1a, 1b and 1c).

Figure 2 (with figures 2 a and 2 b) shows two embodiments for arranging a heating coil in the vicinity of a chamber in a device.

Figure 3 shows a very flat device with a flat chamber.

FIG 4 shows the dependency of particle size vs. needed power to reach a particle temperature of 95 °C with a total mass of 5 mg of particles in water kept constant at 50°C.

Figure 5 shows the basic principle of the invention, exemplified on a single particle.

Summary of the invention

A first subject of the invention is a method for amplification of nucleic acids comprising providing a mixture of a liquid containing the nucleic acids and solid particles having a higher absorption capability for a first energy source than the liquid, said mixture containing the reagents necessary for amplifying the nucleic acids, and introducing said first energy in pulses of between 0.001 and 1 sec for a time sufficient to achieve heating of said solid particles to a temperature of more than 90°C.

A second subject of the invention is a method for heating a liquid comprising providing said liquid and solid particles in a vessel and heating said mixture, wherein said mixture is simultaneously or/and consecutively heated by different kinds of heat sources.

Another subject of the invention is a system for heating a mixture containing a device containing one or more chambers for containing a mixture and a heating source of a first kind and a heating source of a second kind, wherein said heating sources are situated in said system to be effective to heat contents of said chamber of said device when placed in a receptacle for said device.

A further subject of the invention is an apparatus for heating one or more mixtures comprising a receptacle for receiving a device containing a chamber for containing said mixture, a first heat source, and a second heat source, wherein said first and second heat sources are situated in said apparatus to be effective to heat contents of said chamber of said device when placed in said receptacle.

Detailed description of the invention

Methods for the amplification of nucleic acids are known. They are intended to create a large amount of nucleic acids based upon the initial presence of a target nucleic acid serving as a template for the activity of an enzyme capable of replicating a sequence of bases within said target nucleic acid. The replicon itself is used as a target for a replication of a sequence, preferably the sequence of bases that were already subject of the first replication. Thus, a huge number of nucleic acids having an identical sequence are created.

A particularly well established method for the amplification of nucleic acids is the polymerase chain reaction (PCR) method as disclosed in EP 200362. In this method, a reaction mixture is subjected to a repeated cycle of thermal profiles, the temperatures being adapted to effect annealing primers to the target nucleic acid, extending said annealed primer using said target nucleic acid as a template and separating the extension product from its template.

In a first step, a mixture of a liquid containing the nucleic acids and solid particles having a higher absorption capability for a first energy source than the liquid is provided.

The liquid may be any liquid that contains a nucleic acid to be amplified. Furthermore, this liquid contains the reagents necessary for the amplification of the nucleic acids. Those reagents are well known for each amplification method and preferably include an agent for extending a primer, preferably a template dependent DNA- or RNA-polymerase and building blocks that should be attached to the primer for extension, e.g. nucleotides. Furthermore, the mixture will contain reagents useful to establish conditions for the extension reaction, like buffers and cofactors, e.g. salts, of the enzyme used.

Importantly, however, the mixture contains solid particles. Such particles are inert to the conditions for the amplification in that they do not get destructed, particularly at the temperatures used in the method. Those particles are further characterized by their capability to absorb energy from a first energy source better than the liquid. Obviously, the composition of said particle depends upon the energy source.

A first component of a system according to the present invention is a first source providing energy. Preferably, the energy from this first source is transferred in a way without any direct contact between the source of energy and the particles. This kind of transfer of energy is in the following called non-contact energy transfer. Non-contact energy transfer shall be done by electromagnetic radiation, more preferably by electromagnetic radiation in the frequency range of 1 kHz to about 50 GHz most preferred by radio frequency (RF). Particular preferred electromagnetic radiation has a frequency of between 10 kHz and 1 MHz.

The energy from the first energy source is used to specifically heat the particles without directly heating the liquid. This is achieved by using a material having a higher absorption capability for the energy transferred by the first energy source than the surrounding liquid. Particles absorbing energy are preferably selected from the group of solid materials that can absorb radiation in the above mentioned range. Preferred materials are selected from the group of glass particles having additional components to increase electric conductivity, such as metals or metal oxides. More preferred, the material is glass with magnetic pigments distributed therein. Preferably the mass of at least 30%, more preferably at least 50 %, of said solid particles is between 2 pg and 20 ng, preferably between 30 pg and 2 ng. In another mode to define the particles, particularly in case of substantially spherical particles, the diameter of at least 30 %, more preferably of more than 50 %, of the particles is preferably between 0.1 and 100 $\mu$m, more preferably between 0.5 and 10 $\mu$m. Preferably, the average diameter of said particles is between 1 and 10 $\mu$m, more preferably between 2 and 5 $\mu$m. In yet another mode of defining the particles of the invention, at least 30 %, more preferably of more than 50 %, of the particles have a ratio of between 2E8 mm$^{-2}$ and 2E2 mm$^{-2}$, more preferably between 5E5 mm$^{-2}$ and 8E4 mm$^{-2}$, for the diameter to the volume of the particles.

Further preferably, the solid particles have a heat capacity of between 500 and 1000 Jkg$^{-1}$K$^{-1}$, more preferred between 600 and 800 Jkg$^{-1}$K$^{-1}$.

Further preferred, the solid particles are contained in said mixture in between 0.1 and 20 weight-%, more preferred between 0.5 and 5 weight-%.

Preferably, the solid particles have the additional property to be capable of binding nucleic acids. This can be achieved either by unspecific absorption or by specific binding. Unspecific binding of nucleic acids is commonly known to occur at glass surfaces. Thus, particles with a glass surface are preferred. Specific binding can occur if nucleic acids having a sequence complementary to the nucleic acid to be bound to the surface of the particle. Methods for binding, particularly covalent binding of oligonucleotides to surfaces are well known.

In one mode of the invention, the solid particles have at least one primer having a first sequence bound to their surface. Primers are compounds extendable at their 3'-terminus by a polymerizing agent and extension blocks, e.g. mononucleotides using a nucleic acid they are bound to as a template for determination of the sequence.

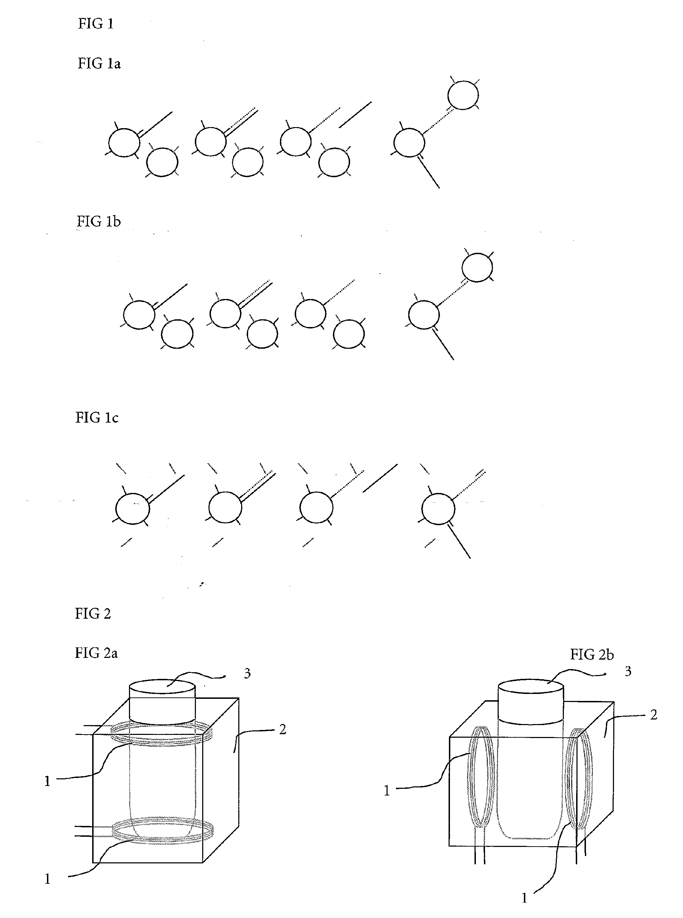

Referring to figure 1, some preferred modes of binding nucleic acids to the particle are explained. In a first mode, shown in figure 1a, a first amount of first particles has bound first primers and a second amount, same or different, of second particles has bound second primers to it. In a second mode, shown in figure 1b, the particles have bound both a first primer and a second primer. In a third embodiment, shown in figure 1c, a first primer is bound to the particles, and a second primer is dissolved in the liquid.

The invention uses the energy of introducing said first energy source in pulses of between 0.001 and 1 sec for a time sufficient to achieve heating of said solid particles to a temperature of more than 90°C. Introducing energy in pulses can be made by switching on the source of energy and directing the energy to the mixture for a specific time. This time is sufficient to heat the particle. The invention uses the capability of the solid particle to in turn pass some heat to the liquid immediately surrounding the particle. The extension of the liquid getting heated by the particle depends upon the length of the pulse. Generally, longer pulses will increase the amount of fluid heated, and shorter pulses will only heat smaller amounts of liquid. Further, the heat-transfer coefficient from the particle to the liquid will determine the heat provided to the liquid. The higher the heat-transfer coefficient, the larger the amount of heat passed. Further, the flow characteristics of the liquid will determine the amount of heat passed. The more convection is made in the liquid, the larger the amount of heat passed, but also the lower the temperature present in the liquid immediate surrounding the particle.

Preferably, the layer being heated to about 90°C is in the present invention around 100 nm thick. The layers farther away from the particle may be heated, but not up to that temperature. The temperature of more than 90°C is needed for providing conditions under which double stranded nucleic acids denature, i.e. singles strands are formed. This is a requirement for annealing of new primers to the extension product and the target nucleic acid.

An essential feature of the invention is that the temperature of the particle and thus of the liquid immediately surrounding the particle is higher than 90°C. On the other side, the temperature should not exceed the boiling temperature of the liquid, at ambient pressure 100°C. The energy needed to heat the particles to that temperature can be determined easily by a few experiments, varying the pulse length and measuring the temperature, further considering whether the liquid shows boiling bubbles.

The method according to the invention has different requirements for the embodiments where primer is bound to the particles compared to the embodiment where the nucleic acids get directly immobilized to the surface. In case of only immobilized primers (cases of figure 1a and 1b), the primer extension step on the surface yields in an extension product being bound to the particle, said product in addition having bound by hybridization the original template. Heating the particle surface and the layer to a temperature of about 95°C will dehybridize the template from the extension product. The template will float from the surface to the part of the fluid outside the layer. Only when and if the temperature of the layer is reduced to a temperature below the melting point of the respective hybrid, the template may bind to nucleic acids on the surface, e.g. another immobilized primer molecule or to a complementary extension product. This can occur in periods of time, where the particle is not heated (outside the energy pulse).

In the embodiment where only one kind of primer is bound to the surface, after denaturation of the hybrid of the extension product and the original template and reduction of the temperature to annealing temperature, the extension product can bind a dissolved primer having moved to the surface. The primer gets extended and the cycle can be repeated. The template can again bind a primer immobilized to the surface which can be extended under annealing conditions.

In embodiments where no immobilized primers are used, any hybrids between primer and template will be formed in the part of the liquid not in the layer and extension will occur. When and if the hybrid of extension product and template enters the layer, denaturation will occur, and the extension product and the template after reduction of the temperature, either by ending the heat pulse or by floating to the part outside the layer can hybridize to respective primers.

The part of the liquid not being located within said layer around said particles is kept at a temperature substantially lower than the temperature within the layer. Preferably, particularly useful for embodiments of the invention wherein primers are dissolved in the residual part of the liquid, the part of the liquid outside the layer is kept at a temperature required for annealing and / or extension of the primer. Therefore, a preferred mode of the invention comprises keeping the liquid at a substantially constant temperature of between 50 and 60 °C.

This keeping the temperature at a constant level may require heating or / and cooling the mixture, preferably the liquid. Whether the liquid needs heating or cooling is dependent upon the amount of energy introduced into the particles and further provided by the particles to the surrounding liquid. If only small amount of energy is passed into the liquid, the liquid may need heating to the appropriate level. This may be the case, when there is low convection within the mixture (no mixing of the particles within the mixture). To the contrary, if the mixture is stirred, the mixture may need cooling to reach the desired temperature in the mixture.

Means for heating the part of the liquid not contained in the layer are known. They can preferably be selected from the group consisting of thermoelectric heating, resistance heating, and fluid mediated heating. Appropriate heat sources are known, for example from conventional thermal cyclers. Particularly preferred heat sources are resistance heaters. In those, electric current is used to heat a heater which is in contact with a block, e.g. made from aluminum, in which the vessel containing the mixture is contained.

Means for cooling are also well known. They may include fluid cooling, e.g. by directing a flow of gas or liquid to the vessel or a block containing said vessel, or by thermoelectric cooling realized with Peltier elements for example.

The method according to the invention may even preferably include both cooling and heating during the course of the same amplification; e.g. in the beginning, when not too much heat was supplied by the heating of the particles, heating may be needed. In later stages of the method, the amount of heat passed from the particles may not require independent heating of the liquid, and even may require cooling. This problem can easily be solved by using a sensor to measure the temperature within the liquid or close to the liquid e.g. in the block containing the vessel and to control the temperature by a computer program.

In a very preferred mode, therefore, the present invention comprises two different modes of heating and one mode of cooling; said heating and cooling process being controlled and regulated by a computer program dependent upon the temperature of the liquid.

In a broader sense, the invention is directed to a method for heating a mixture of a liquid and particles comprising providing said mixture in a vessel and heating said mixture, wherein said mixture is simultaneously or/and consecutively heated by different kinds of heat sources.

A vessel according to invention is a container for keeping the mixture under the conditions of the method. Thus, the container should be heat resistant, resistant to radiation of the amount and kind provided to the mixture, be resistant to the reagents contained in the mixture and be tight so that the mixture cannot escape the container.

Preferably, at least one of said methods of heating is selected from the group consisting of inductive heating, thermoelectric heating, resistance heating, radiative heating and fluid mediated heating.

Inductive heating is performed by the application of electromagnetic radiation with a frequency / wave length of between 10 kHz and 1 MHz. Thermoelectric heating is achieved by a source known as Peltier element which develops heat by the well known Peltier effect.

Resistance heating uses the effect that the resistance of small diameter wires upon current flow leads to a loss of energy by heat.

Radiative heating is created by directing radiation to the mixture in the vessel, said radiation having a wavelength absorbed by the mixture or the vessel and transferring said radiation to heat energy.

Fluid mediated heating is performed by directing a flow of a fluid, i.e. a liquid or a gas, to the vessel or the mixture, said fluid having a temperature being higher than the temperature of the mixture. The higher the difference in temperature between the fluid and the mixture, the quicker the mixture gets heated. Further, the flow characteristics of the fluid will determine the amount of heat passed.

Preferably, said first heating is done by non-contact heating and the second heating is done by contact heating. Contact heating is heating wherein the hot medium contacts the material to be heated, such that energy can flow through the contact surface between them from the heating medium to the material. As the particles in the mixture are dispersed within the mixture, heating of the particles within the mixture to a temperature above the temperature of the surrounding liquid is not feasible using contact heating. However, heating of the part of the liquid outside the layer around the particles is quite feasible by contact heating.

Non-contact heating is performed if the source of the energy cannot directly contact the material to be heated. Thus, this mode of heating is preferred for heating the particles within the mixture, without substantial direct heating the part of the liquid outside the layer. The most preferred mode of non-contact heating is heating by radiation, e.g. by radiation that is not absorbed by the part of the liquid not in the layer surrounding the particles.

Further the modes of heating can be distinguished by the medium of transport of the heat. Preferably, said liquid is heated by a combination of inductive and resistance heating.

Very preferred, the present invention is concerned with a method wherein said heating is performed using a combined heating source. Such heat source is applicable for both, contact heating as well as non-contact heating and is as such especially suited for applications in which both methods are used at the same time.

The heating source according to this invention is a combination of a resistive heater and a coli for inductive heating. A preferred design is a heating coil with a defined resistance for resistive heating which is designed in a way that it is able to generate an electromagnetic field for inductive heating as soon as it is driven with an appropriate alternating current of an appropriate frequency. The coil can be formed by a wire or it can be designed in another way e.g. on a printed circuit board or as conductor of any kind of material on a substrate like ceramic or polyimide. One other option is that the coil is formed by Thin- or Thickfilm technology on a suitable substrate. The coil can be located below, on top or at the sides of the receptacle or even surround the device in a way that the device is inside the coil depending on the design of the coil. The coil should be designed in a way that it produces the strongest magnetic field which is possible. The coil can be part of an oscillator circuit (parallel resonant circuit) which is driven by a high frequency generator at the resonant frequency of the circuit to generate a strong magnetic field.

The material for the receptacle which is heated by contact heating through the combined heating element has to have a high thermal conductivity to achieve a good heat transfer from the heater to the device containing the chamber for the mixture. Furthermore, the material has to be inert to electromagnetic fields meaning that it should not influence the electromagnetic field for non contact heating in a negative way. Preferably, the material for the receptacle is selected from the group of materials with zero electrical conductivity. More preferably the material is selected from the group of ceramic materials (e.g. AIN ceramic).

For resistive heating the heater needs to be in contact with the receptacle for the device containing the mixture in a chamber (contact heating). For the inductive heating of the solid particles and the layer around the particles, the combined heater needs to be arranged in an appropriate distance to the device with the chamber containing the mixture (non-contact heating). The most preferred design is a flat coil on a substrate which can be contacted to the receptacle for contact heating and is located near enough to the chamber containing the mixture to have the mixture in the center of the generated electromagnetic field for non-contact heating of the solid particles.

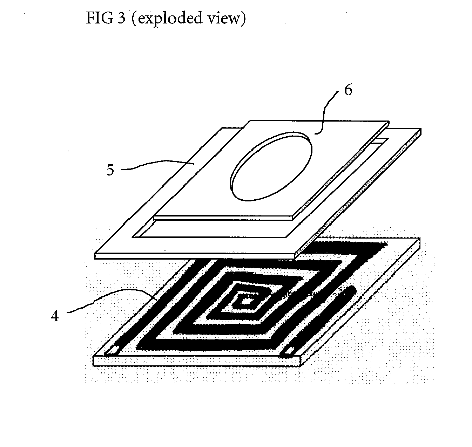

FIG 2a and FIG 2b show possible arrangements of a combined heating element with two coils formed by a wire (1), a receptacle (2) and a device with a chamber (3).

FIG 3 shows in exploded view a possible arrangement of a combined heating element carried out as flat coil on a suitable substrate (4) with a receptacle (5) and a flat device with a chamber (6).

In a preferred mode of the invention, similar to the situation as discussed above for the method for amplification of nucleic acids, the method further comprises active cooling of said liquid.

Another embodiment of the invention is an apparatus for heating one or more liquids comprising a receptacle for receiving a vessel containing a chamber for containing a liquid, a first heat source, and a second heat source, said first and second heat sources being situated in said apparatus to be effective to heat contents of said chamber of said vessel when placed in said receptacle.

Another embodiment of the invention is a system for heating a liquid containing a vessel containing one or more chambers for containing a liquid and a heat source of a first kind and a heat source of a second kind, wherein said heat sources are situated in said system to be effective to heat contents of said chamber of said device when placed in a receptacle for said vessel.

Such systems are useful for the methods as disclosed above. Conveniently, the system has a housing comprising said heat sources, particularly preferred isolated such that heat developed by the sources is mainly directed to the vessel and not to the environment. This is particularly important for radiation sources, also for health reasons.

As discussed above, a system is preferred wherein said first heat source is a non-contact heat source and the second heat source is a contact heat source.

Examples

Example 1

Treating a liquid containing solid particles by radiation

A model experiment with mixtures of the following components was created and carried out in the lab:

| Component | Characteristic | amount |
|---|---|---|
| Magnetic glass particles | Size 0.5 - 5 $\mu$m<br>Dry particles (not suspended) like commercially available from various vendors | Sample 1: 12 mg / mL<br>(1.2 weight-%) Sample 2: 127mg/mL<br>(12.7 weight-%) |
| De-ionized water | | 1 mL |

In the model experiment the two samples were heated by induction heating in polypropylene tubes from room temperature to an end temperature of about 100 °C. The tubes were placed inside a three turn induction heating coil. The experiment was carried out using different power supplies as energy sources working at different frequencies. Best results were obtained with a power supply working at around 200 kHz and providing 20 kW of electrical power. The power was continuously switched on during the experiment until the sample reached the end temperature. The temperature was measured by a thermocouple.

The following table gives an overview over the results from this experiment.

| Experiment | Starting temperature | End temperature | Time needed |
|---|---|---|---|
| Sample 1 | 22 °C | 100 °C | 30 seconds |
| Sample 2 | 22 °C | 100 °C | 5 seconds |

This experiment shows that it is generally possible to heat solid particles in an adequate volume of an aqueous solution suitable for the analysis of samples in health care by non contact induction heating. In this experiment the complete liquid in which the solid particles were suspended was heated up to the end temperature thus a lot more energy was required in that experiment than if only the solid particles and the surrounding layer have to be heated to the end temperature.

Example 2

Theoretical experiment / model simulation

With a simple model the energy input into one single particle $Q_{in}$ through non contact heating and the occurring energy loss to the surrounding, colder liquid phase $Q_{out}$ was simulated (see FIG 5).

The model was set up for one single bead. The first input into the model is the total power which is brought in by non contact heating. This total power is later divided trough the calculated number of particles to get the input power per a single bead. The second input is the particle size influencing the number of particles at a given total mass of solid particles as well as the surface area of the solid particles. The third input value is the total mass of all particles. The number of particles is calculated as follows:

$$Number\_of\_Particles = \frac{m_{MGP}}{m_{1MGP}}$$

with

$m_{MGP}$ = mass of all particles in the solution e.g 5 mg $\qquad$ [kg]

$m_{1MGP}$ = mass of one single particle $\qquad$ [kg]

The mass of one single particle is calculated by

$$m_{1MGP} = \rho_{MGP} * V_{MGP} \quad \text{with} \qquad [kg]$$

$\rho_{MGP}$ = specific density of the particles $\qquad$ [kgm$^-$$^3$]

$V_{MGP}$ = volume of one particle $\qquad$ [m$^3$]

The volume of one particle results from

$$V_{MGP} = \frac{4}{3}\pi\left(\frac{d_{bead}}{2}\right)^3 \quad \text{with} \qquad [m^3]$$

$$d_{bead} \qquad = \text{diameter of one bead e.g. } 2.5\ \mu m \qquad\qquad [m]$$

The model calculates stepwise for a given time interval $\Delta t$ e.g. 0.001 seconds per step.

The bead is regarded as container with an input of energy $Q_{in}$ and an output of energy $Q_{out}$. The change of energy from the time t-1 to the time t is calculated by

$$\Delta Q_{bead_t} = \Delta Q_{in_t} - \Delta Q_{out_t} \qquad\qquad [Ws]$$

The input energy from induction heating is the product from the input power and the time interval.

$$\Delta Q_{in_t} = P_{in\_per\_bead_t} * \Delta t \qquad\qquad [Ws]$$

The energy in the bead at the time t is calculated by

$$Q_{bead_t} = \sum_{t=0}^{t=t} \Delta Q_{bead_t} \qquad\qquad [Ws]$$

The change in bead temperature from time t -1 to time t results from

$$\Delta T_{bead_t} = \frac{\Delta Q_{bead_t}}{m_{1bead} * c_{bead}} \qquad\qquad [K]$$

which is added to the actual bead temperature as follows

$$T_{bead_t} = T_{bead_{t-1}} + \Delta T_{bead_t} \qquad\qquad [^{\circ}C]$$

On the output side, the energy loss trough heat transfer into the liquid phase is

$$\Delta Q_{out_t} = P_{out\_per\_bead_t} * \Delta t \qquad\qquad \text{with} \qquad\qquad [Ws]$$

$$P_{out\_per\_bead_t} = (T_{bead_t} - T_{liquid}) * \alpha * A \qquad\qquad [W]$$

$\Delta Q_{bead\_t}$ = change of total energy in the bead during time interval $\Delta t$ [Ws]

$\Delta Q_{in\_t}$ = energy input into the bead during time interval $\Delta t$ [Ws]

$\Delta Q_{out\_t}$ = energy output from the bead during time interval $\Delta t$ [Ws]

$P_{in\_per\_bead\_t}$ = power input into one bead at time t [W]

$\Delta t$ = time interval from time t-1 to time t [s]

$Q_{bead\_t}$ = total energy in the bead at time t [Ws]

$\Delta T_{bead\_t}$ = temperature change of bead during time interval $\Delta t$ [K]

$m_{1bead}$ = mass of one single bead [kg]

$c_{bead}$ = specific heat capacity of the bead material [$WsKg^{-1}K^{-1}$]

$T_{bead\_t}$ = temperature of bead at time t [°C]

$P_{out\_per\_bead\_t}$ = power output from one bead at time t [W]

$T_{liquid}$ = temperature of liquid phase around bead (constant) [°C]

$$\alpha \qquad = \text{heat-transfer coefficient from bead to liquid} \qquad [\text{Wm}^{-2}\text{K}^{-1}]$$

$$A \qquad = \text{surface area of one single bead (spherical)} \qquad [\text{m}^2]$$

The temperature of the surrounding liquid phase liquid was considered to stay constant at a temperature of 50°C. To fulfill this assumption the surrounding liquid phase would have to be cooled by suitable means. At the beginning of the simulation the bead has the same temperature as the liquid phase.

Out of the results from the model experiment carried out in the lab, the total input power was fed into the model. While changing the size of the solid particles and the total amount of solid particles in the solution, the theoretically reachable temperature with a given input power can be calculated.

Results

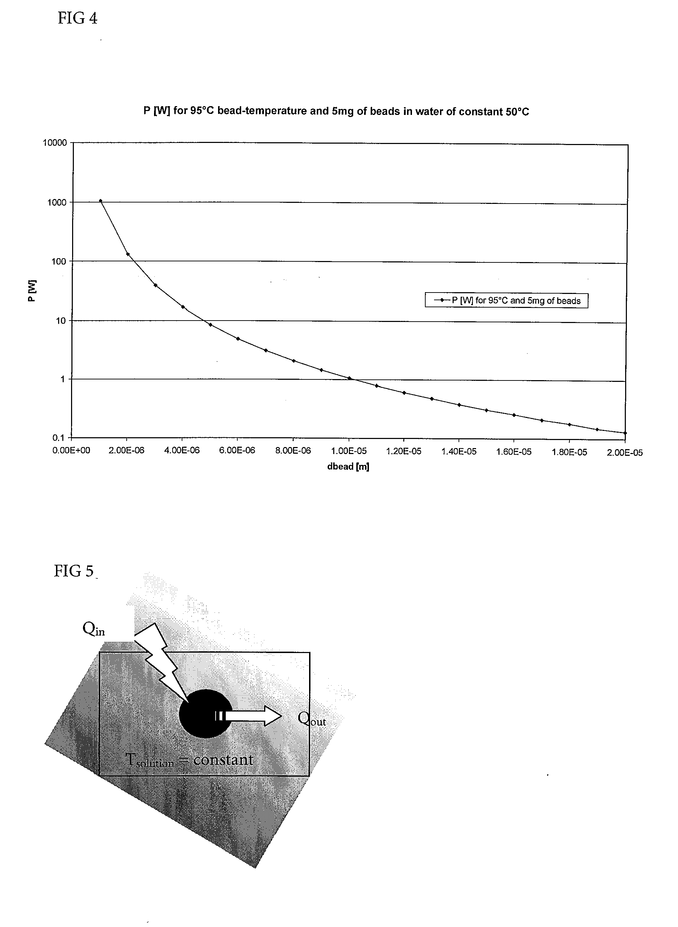

The following paragraph sums up the results from a simulation based on the model described above. FIG 4 shows the dependency between particle size and needed power to get 5 mg of solid particles of a specific size heated up to a temperature of 95 °C in water which is kept at a constant temperature of 50 °C.

The needed power shown in FIG 4 is only the power which has to be coupled into the beads to reach the target temperature. The total power needed by the system to get the same effect is dependant on the energy efficiency of the total system. The generator for the electromagnetic field in example 1 was providing 20 kW of electrical power resulting in approximately 70 W of coupled power into the system heating up the beads and the liquid they were suspended in.

Reference numerals

1    Heating wire

2    Receptacle

3    Device with chamber

4    Flat coil

5    Receptacle

6    Flat device with chamber

**Claims**

1.  A method for amplification of nucleic acids comprising

    a) providing a mixture of a liquid containing the nucleic acids and solid particles having a higher absorption capability for a first energy source than the liquid, said mixture containing the reagents necessary for amplifying the nucleic acids,
    b) introducing said first energy in pulses of between 0.001 and 1 sec for a time sufficient to achieve heating of said solid particles to a temperature of more than 90°C,

    **characterized in that** the liquid is actively kept at annealing and extension temperature.

2.  The method of claim 1 wherein said particles have an average diameter of between 0.1 and 100 $\mu$m.

**3.** The method of any of claims 1 and 2, wherein said solid particles are nucleic acid binding solid particles.

**4.** The method of any of claims 1 to 3, wherein said first energy source is an electromagnetic field.

**5.** The method of any of claims 1 to 4, wherein said energy is radiation energy of a frequency of between 1kHz and 50 GHz.

**6.** The method of any of claims 1 to 5, wherein the mass of at least 30% of said solid particles is between 2 pg and 20 ng.

**7.** The method of any of claims 1 to 6, wherein said solid particles have a heat capacity of between 500 and 1000 $Jkg^{-1}K^{-1}$.

**8.** The method of any of claims 1 to 7, wherein said solid particles are contained in said mixture in between 0.1 and 20 w-%.

**9.** The method of any of claims 1 to 8, wherein said solid particles have primers bound to their surface.

**10.** The method of any of claims 1 to 9, further comprising keeping the liquid at a substantially constant temperature of between 50 and 60 °C.

**11.** A method for heating a liquid comprising providing a mixture of said liquid and solid particles in a vessel and heating said mixture,
**characterized in that** said liquid is simultaneously or/and consecutively heated by different kinds of heat sources.

**12.** The method according to any of claims 1 to 11, wherein at least one of said methods of heating is selected from the group consisting of

- inductive heating,
- thermoelectric heating,
- resistance heating,
- radiative heating and
- fluid mediated heating.

**13.** The method according to any of claims 1 to 12, wherein said mixture is heated by a combination of inductive and resistance heating.

**14.** The method according to any of claims 1 to 13, wherein said first heating is done by contact heating and the second heating is done by non-contact heating.

**15.** The method according to any of claims 1 to 14, wherein said heating is performed using a combined heating source.

**16.** The method according to any of claims 1 to 15, wherein said method includes inductively heating said solid particles dispersed in said liquid.

**17.** The method according to any of claims 1 to 16, further comprising active cooling of said mixture.

**18.** A system for heating a mixture containing

- a device containing one or more chambers for containing a mixture and
- a heating source of a first kind and
- a heating source of a second kind,

wherein said heating sources are situated in said system to be effective to heat contents of said chamber of said device when placed in a receptacle for said device.

**19.** The system of claim 18, wherein said first heating source is a contact heating source and the second heating source is a non-contact heating source.

**20.** An apparatus for heating one or more mixtures comprising

- a receptacle for receiving a device containing a chamber for containing a mixture,
- a first heat source, and
- a second heat source,

said first and second heat sources being situated in said apparatus to be effective to heat contents of said chamber of said device when placed in said receptacle.

FIG 1

FIG 1a

FIG 1b

FIG 1c

FIG 2

FIG 2a

FIG 2b

FIG 3 (exploded view)

FIG 4

P [W] for 95°C bead-temperature and 5mg of beads in water of constant 50°C

FIG 5

**European Patent Office**

**EUROPEAN SEARCH REPORT**

Application Number

EP 05 10 6980

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | US 2004/129555 A1 (MARCHITTO KEVIN S ET AL) 8 July 2004 (2004-07-08) * paragraph [0118]; figure 6b * ----- | 1-20 | C12Q1/68 H05B6/80 H05B6/02 H05B6/10 H05B3/78 |
| A | WO 95/15671 A (INCELTEC LTD; RAY, RONNIE, AURUN; WAKEFIELD, ANDREW, JEREMY) 8 June 1995 (1995-06-08) * page 10, line 16 - line 31 * ----- | 1-20 | |
| D,A | US 2002/061588 A1 (JACOBSON JOSEPH M ET AL) 23 May 2002 (2002-05-23) * paragraphs [0051], [0052] * ----- | 1-20 | |
| A | DE 100 36 486 A1 (BIOTIX GMBH) 14 February 2002 (2002-02-14) * paragraph [0033] - paragraph [0035] * ----- | 1-20 | |
| A | US 5 643 764 A (KOSAK ET AL) 1 July 1997 (1997-07-01) * column 14, line 35 - column 15, line 12 * ----- | 1-20 | |

TECHNICAL FIELDS SEARCHED (IPC)

C12Q
H05B

The present search report has been drawn up for all claims

| Place of search | Date of completion of the search | Examiner |
|---|---|---|
| Munich | 18 January 2006 | Gea Haupt, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 748 081 A1**

## ANNEX TO THE EUROPEAN SEARCH REPORT
## ON EUROPEAN PATENT APPLICATION NO.

EP 05 10 6980

18-01-2006

| Patent document cited in search report | | Publication date | Patent family member(s) | | Publication date |
|---|---|---|---|---|---|
| US 2004129555 | A1 | 08-07-2004 | US | 6673214 B1 | 06-01-2004 |
| | | | US | 2004086957 A1 | 06-05-2004 |
| WO 9515671 | A | 08-06-1995 | AU | 1194995 A | 19-06-1995 |
| US 2002061588 | A1 | 23-05-2002 | NONE | | |
| DE 10036486 | A1 | 14-02-2002 | AU | 8569101 A | 05-02-2002 |
| | | | WO | 0208455 A2 | 31-01-2002 |
| | | | EP | 1305448 A2 | 02-05-2003 |
| US 5643764 | A | 01-07-1997 | AU | 661768 B2 | 03-08-1995 |
| | | | AU | 3619193 A | 03-09-1993 |
| | | | CA | 2130108 A1 | 19-08-1993 |
| | | | EP | 0626017 A1 | 30-11-1994 |
| | | | WO | 9316200 A1 | 19-08-1993 |
| | | | US | 5550044 A | 27-08-1996 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0201184 A
- EP 0200362 A
- US 20020061588 A
- US 20040129555 A
- US 6633785 B
- EP 200362 A